# EUROPÄISCHE PATENTANMELDUNG

(11) **EP 1 795 535 A1**
(43) Veröffentlichungstag der Anmeldung: **13.06.2007**
(21) Anmeldenummer: 05026889.5
(22) Anmeldetag: 08.12.2005
(51) Int. Cl.: C07H 13/04

(54) **Verfahren zur Herstellung einer stabilen Lösung von 1,3,4,6-Tetra-O-acetyl-2-O-trifluormethansulfonyl-beta-D-manno-pyranose (Mannosetriflat)**

(71) Anmelder: ABX advanced biochemical compounds Biomedizinische Forschungsreagenzien GmbH, 01454 Radeberg (DE)
(72) Erfinder: Müller, Marco, 28876 Oyten (DE); Niegel, Harald, 01640 Coswig (DE); Bonsen, Eva Maria, 01099 Dresden (DE); Hoepping, Alexander, 01099 Dresden (DE)
(74) Vertreter: Carlsohn, Alexander

(57) **Zusammenfassung**

Die Erfindung betrifft ein Verfahren zur Herstellung einer lagerstabilen Lösung einer Verbindung gemäß Formel (I) 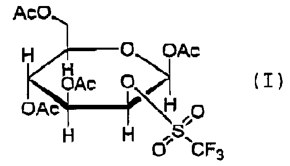 in einem Lösungsmittel, das aus der Gruppe gewählt ist, die Acetonitril, deuteriertes Acetonitril sowie Gemische dieser umfaßt. Dabei ist vorgesehen, daß die Verbindung eine Reinheit von zumindest 99,0 Gew.-% aufweist, wobei der Anteil an Trifluormethansulfonsäure 0,2 Gew.-% nicht übersteigen darf, das Lösungsmittel eine Reinheit von 99,9 Gew.-% aufweist, wobei der Wassergehalt des Lösungsmittels 0,05 Gew.-% nicht übersteigen darf; und das Lösen und Filtrieren bei einer Luftfeuchtigkeit von höchstens 60 % und einer Raumtemperatur von höchstens 30 °C durchgeführt wird.

## Beschreibung

Die Erfindung betrifft ein Verfahren zur Herstellung von stabilen Lösungen von 1,3,4,6-Tetra-*O*-acetyl-2-*O-*trifluormethansulfonyl-β-D-mannopyranose (Mannosetriflat). Sie betrifft ferner eine mittels dieses Verfahrens erhaltene Mannosetriflat-Lösung.

1,3,4,6-Tetra-*O*-acetyl-2-*O*-trifluormethansulfonyl-β-D-manno-pyranose (Mannosetriflat) ist ein Ausgangsstoff für die Herstellung von 2-[¹⁸F]Fluor-2-desoxy-D-glucose (FDG), das als Radiopharmakon in der Positronen-Emissions-Tomographie (PET) eingesetzt wird.

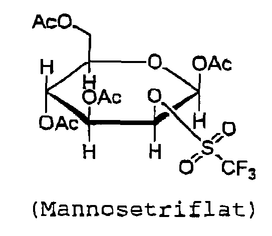

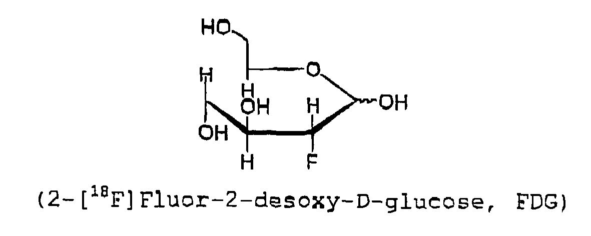

Die Positronen-Emissions-Tomographie ist eine Methode, mit der pathologische Prozesse im menschlichen Organismus auf molekularer Ebene sichtbar gemacht werden können. Dabei wird dem Patienten eine mit einem radioaktiven Isotop markierte Verbindung injiziert und die Verteilung der Aktivität mittels einer PET-Kamera gemessen. Solche Isotope sind u. a. F-18 und C-11. 2-[¹⁸F]Fluor-2-desoxy-D-glucose (FDG) ist gegenwärtig das am meisten genutzte Radiopharmakon.

Die Herstellung von FDG erfolgt nach der Methode von Hamacher (K. Hamacher, H. H. Coenen, G. Stöcklin, J. Nucl. Med. 1986, 27, 235-238). Dabei wird Mannosetriflat als Präkursor in Gegenwart von Kryptofix [2.2.2] mit ¹⁸F-Fluorid umgesetzt und das erhaltene Zwischenprodukt 2-[¹⁸F]-Fluor-2-deoxyglucosetetraacetat entweder sauer oder basisch verseift. Dieses Verfahren wird gegenwärtig in sogenannten Synthesemodulen für die Versorgung mit FDG eingesetzt. Das *Synthesemodul* Tracerlab MX der Firma General Electric (GE), arbeitet mit 20 bis 60 mg Mannosetriflat als Präkursor, der unmittelbar vor Synthesebeginn in einem Lösungsmittel gelöst wird. Die spezifische Aktivität des erhaltenen FDG beträgt zwischen 2 und 5 Ci/µmol. Die spezifische Aktivität von 2 Ci/µmol ¹⁸F-Fluorid entspricht ungefähr einer Stoffmenge von 1 nmol. Zur Erhöhung der spezifischen Aktivität ist es daher notwendig, den Einsatz von Präkursor und Lösungsmittel zu minimieren. Die Mikrofluidik und die Mikroreaktortechnik bieten einen Ansatz dazu. Optimale Reaktionsbedingungen (laminarer Fluß, hervorragende thermische Durchmischung, schnelle Reaktionen) führen zu einer drastischen Erhöhung von Ausbeuten, im speziellen Fall der Radioausbeute und einer Verringerung der Zahl von Nebenprodukten. Idealerweise sollte die Portionierung und Dosierung von Präkursoren im µg-Maßstab erfolgen. Damit ist man zwar immer noch um einen Faktor von 1000 über den tatsächlich notwendigen Stoffmengen, nähert sich aber den Idealbedingungen schon stark an. Eine exakte Portionierung von µg-Mengen als Feststoff ist technisch nur schwer zu realisieren. Vielmehr sollte der Einsatz von vorportionierten Präkursoren in geeigneten Lösungsmitteln eine exakte Dosierung für den Einsatz im Mikroreaktor erlauben. Idealerweise werden mg-Mengen des Präkursors in µl-Mengen Lösungsmitteln zudosiert.

Als Lösungsmittel für Mannosetriflat wird Acetonitril oder deuteriertes Acetonitril verwendet. Es ist jedoch bekannt, daß Lösungen von Mannosetriflat nur kurze Zeit bei Raumtemperatur stabil sind. Deshalb werden Lösungen von Mannosetriflat in Acetonitril oder deuteriertem Acetonitril erst unmittelbar (maximal ein bis zwei Stunden vor der Umsetzung zu FDG hergestellt werden.

Aus Stabilitätsstudien, die von ABX mit Mannosetriflat durchgeführt wurden, ist ferner bekannt, daß die Lagerstabilität des Feststoffs bei Lagerung im Tiefkühlschrank (-20 t 5 °C) nur mit zwei Jahren angegeben werden kann.

Um die Vorteile der Mikrofluidik und der Mikroreaktortechnik nutzen zu können, ist es jedoch dringend erforderlich, Präkursoren bereits gelöst zur Verfügung zu stellen, und zwar unabhängig vom Synthesebeginn, um eine exakte Dosierung des Präkursors zu ermöglichen. Aber auch für eine weitere Automatisierung des Herstellungsprozesses von ¹⁸F-FDG mittels herkömmlicher Synthesemodule wäre eine Bereitstellung von bereits gelöstem Mannosetriflat wünschenswert. Aus dem Stand der Technik ist jedoch kein Verfahren bekannt, mit dem eine lagerstabile Lösung einer vorgegebenen Menge an Mannosetriflat in einer vorgegebenen Menge an Acetonitril hergestellt werden kann. Es ist daher bisher nicht möglich, eine portionierte Menge gelösten Mannosetriflats herzustellen, zu konfektionieren und auszuliefern, so daß eine automatisierte Herstellung von FDG in Mikroreaktoren nicht möglich ist.

Aufgabe der Erfindung ist es, die Nachteile nach dem Stand der Technik zu beseitigen. Es soll insbesondere ein Verfahren angegeben werden, mit dem lagerstabile Lösungen von Mannosetriflat in Acetonitril und deuteriertem Acetonitril hergestellt werden können. Ferner soll eine lagerstabile Lösungen von Mannosetriflat in Acetonitril und deuteriertem Acetonitril angegeben werden, die mittels dieses Verfahrens erhalten werden kann.

Diese Aufgabe wird durch die Merkmale der Ansprüche 1, 11 und 12 gelöst. Zweckmäßige Ausgestaltungen der Erfindungen ergeben sich aus den Merkmalen der Ansprüche 2 bis 10 sowie 13.

Nach Maßgabe der Erfindung ist ein Verfahren zur Herstellung einer Lösung einer Verbindung gemäß Formel (I) 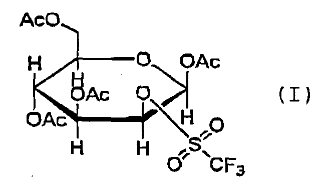 in einem Lösungsmittel vorgesehen, das aus der Gruppe gewählt ist, die Acetonitril, deuteriertes Acetonitril sowie Gemische dieser umfaßt, wobei das Verfahren die Schritte
(a) Bereitstellen der Verbindung gemäß Formel (I), mit einer Reinheit von zumindest 99,0 Gew.-%, wobei der Anteil an Trifluormethansulfonsäure 0,2 Gew.-% nicht übersteigen darf;
(b) Bereitstellen des Lösungsmittels mit einer Reinheit von 99,9 Gew.-%, wobei der wassergehalt des Lösungsmittels 0,05 Gew.-% nicht übersteigen darf;
(c) das Lösen der Verbindung gemäß Formel (I) in dem Lösungsmittel bei einer Luftfeuchtigkeit von höchstens 60 % und einer Raumtemperatur von höchstens 30 °C;
(d) das Mikrofiltrieren der in schritt (c) erhaltenen Lösung über einem Mikrofilter mit einer Porengröße von höchstens 0, 3 µm; und
(e) das Abfüllen der in Schritt (c) erhaltenen Lösung in zumindest einen Behälter aus einem inerten Material und Verschließen des Behälters mit einem Verschluß aus demselben oder einem anderen inerten Material;
umfaßt.

Bei der Verbindung der Formel 1 handelt es sich um 1,3,4,6-Tetra-*O*-acetyl-2-*O*-trifluormethansulfonyl-β-D-mannopyranose, das auch als Mannosetriflat oder TATM bezeichnet wird. Acetonitril ist eine Verbindung der Formel (II); deuteriertes Acetonitril eine Verbindung der Formel (III), das auch als Deuteroacetonitril bezeichnet wird.

CH₃-C≡N (I)

CD₃-C≡N (II)

In Schritt (a) beziehen sich Angaben in Gew.-% auf das Gesamtgewicht der bereitgestellten Verbindung der Formel (I) und aller in ihr enthaltenen verunreinigungen.

In Schritt (b) beziehen sich Angaben in Gew.-% auf das Gesamtgewicht der bereitgestellten Lösungsmittels und aller in ihm enthaltenen Verunreinigungen.

Es hat sich überraschenderweise gezeigt, daß die durch das erfindungsgemäße Verfahren erhaltene Lösung der Verbindung gemäß Formel (I) in Acetonitril oder deuteriertem Acetonitril bei Temperaturen bis zu 30 °C über ein Jahr lagerstabil war. Dies war nach dem Stand der Technik nicht zu erwarten. Voraussetzung, um diese hohe Lagerstabilität der Lösung zu erreichen, sind die in dem verfahrensschritten (a) bis (e) genannten Bedingungen, insbesondere die hohe Reinheit der bereitgestellten Verbindung gemäß Formel (I) in bezug auf Verunreinigungen mit ähnlicher Struktur und auf fluorierte Verunreinigungen.

Das erfindungsgemäße Verfahren ermöglicht die Lagerung der Lösung bei Raumtemperatur, so daß die bisher erforderliche, aufwendige Lagerung des ungelösten Mannosetriflats in Tiefkühlschränken entfällt und damit auch der Transport von Lösungen des Mannosetriflats in Acetonitril deutlich vereinfacht wird. Das Verfahren ermöglicht die Bereitstellung der Lösung in Ampullen oder Vials, die eine vorgegebene Menge der Lösung bei einer vorgegebenen Konzentration an Mannosetriflat enthalten. Auf diese Weise kann eine vereinfachung der routinemäßigen Herstellung von FDG erreicht werden, was eine Voraussetzung für dessen Herstellung in Mikroreaktoren ist, aber auch zu einer Verbesserung der Herstellung in herkömmlichen Synthesemodulen führt.

Vorzugsweise enthält die in Schritt (a) bereitgestellte Verbindung der Formel I höchstens 0,2 Gew.-% von Verunreinigungen, die zu der Gruppe gehören, die aus 2,3,4,6-Tetra-O-acetyl-β-D-mannopyranose (1-OH-Tetraacetylmannose, 1-OH-TAM), 1,3,4,6-Tetra-*O*-acetyl-β-D-mannopyranose (2-OH-Tetraacetylmannose, 2-OH-TAM) und Gemischen dieser besteht. 1-OH-TAM, 2-OH-TAM sind dem Mannosetriflat strukturell ähnliche Verbindungen, die - wie sich herausgestellt hat - eine Zersetzung des Mannosetriflat in Acetonitril initiieren können. Ferner sollte die in Schritt (a) bereitgestellte Verbindung der Formel I vorzugsweise höchstens 0,25 Gew.-% Cyclohexan, höchstens 0,18 Gew.-% Dichlormethan, höchstens 0,01 Gew.-% Pyridin und höchstens 0,10 Gew.-% Ethanol enthalten.

Das Acetonitril sollte eine Reinheit von mindestens 99,9 % und einen Wassergehalt von höchstens 0,05 Gew.-% aufweisen. Das deuterierte Acetonitril sollte ein Isotopenanreicherung von 99,8 % und einen Wassergehalt von höchstens 0,05 Gew.-% aufweisen.

Schritt (c) sollte in einer Umgebungsluft durchgeführt werden, die höchstens 350.000 Partikel mit einer Größe von 0,5 µm oder größer pro Kubikmeter Umgebungsluft und höchstens 2.000 Partikel mit einer Größe von 5 µm oder größer pro Kubikmeter Umgebungsluft enthält. Dies entspricht den Vorgaben der Reinraumklasse C gemäß der GMP-Richtlinie. Die Konzentration der Lösung wird vorzugsweise auf 0,1 bis 1000 mg/ml eingestellt.

Die Mikrofiltration gemäß Schritt (d) sollte zweckmäßigerweise in einer Umgebungsluft durchgeführt werden, die höchstens 3500 Partikel mit einer Größe von 0,5 µm oder größer pro Kubikmeter Umgebungsluft und keine Partikel mit einer Größe von 5 µm oder größer pro Kubikmeter Umgebungsluft enthält, durchgeführt werden. Dies entspricht den Vorgaben der Reinraumklasse A gemäß der GMP-Richtlinie.

Nach der Mikrofiltration wird die Lösung unter Schutzgas, beispielsweise Argon oder Stickstoff, in den Behälter gemäß Schritt (e) überführt.

Der Behälter, in denen die Lösung gemäß Schritt (e) abgefüllt wird, wird vorzugsweise mittels γ-Strahlung sterilisiert, bevor die Lösung in den Behälter gefüllt wird. Der Behälter ist besonders bevorzugt ein Glasbehälter, beispielsweise eine Ampulle, Phiole oder Vial. Das Glas aus dem der Glasbehälter besteht, sollte der hydrolytischen Glasklasse 1 gemäß DIN EN ISO 8362-1 angehören. Der Verschluß des Behälters besteht vorzugsweise aus Chlorbutylgummi. Der Verschluß ist bevorzugt mit einem inerten Material wie beispielsweise Polytetrafluorethylen (PTFE), beispielsweise Flurotec®, ummantelt. Der Vorschluß kann ferner mechanisch an dem Behälter beispielsweise durch Crimpen gesichert werden.

Behälter und Verschluß müssen so gewählt werden, daß keine Feuchtigkeit oder Luft in den Behälter gelangen kann, nachdem dieser verschlossen worden ist. Ferner dürfen aus dem Behälter und dem Verschluß keine Substanzen in die Lösung eintreten, die eine Zersetzung des Mannosetriflat initiieren könnten.

Nach Maßgabe der Erfindung ist ferner einer Lösung der Verbindung gemäß Formel (I) 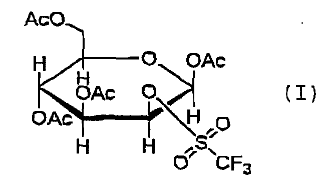 in einem Lösungsmittel, das aus der Gruppe gewählt ist, die Acetonitril, deuteriertes Acetonitril sowie Gemische dieser umfaßt, vorgesehen, die mittels des erfindungsgemäßen Verfahrens hergestellt worden ist.

Die Erfindung wird nachfolgend anhand von Beispielen und Bezugnahme auf die Zeichnungen näher erläutert. Dabei zeigen
Fig. 1 ein ¹⁹F-NMR-Spektrum einer erfindungsgemäßen Lösung, das einen Tag nach dere Herstellung aufgenommen wurde;
Fig. 2 ein ¹⁹F-NMR-Spektrum einer erfindungsgemäßen Lösung, das drei Tage nach deren Herstellung aufgenommen wurde;
Fig. 3 ein ¹⁹F-NMR-Spektrum einer erfindungsgemäßen Lösung, das eine Woche nach deren Herstellung aufgenommen wurde;
Fig. 4 ein ¹⁹F-NMR-Spektrum einer erfindungsgemäßen Lösung, das einen Monat nach deren Herstellung aufgenommen wurde;
Fig. 5 ein ¹⁹F-NMR-Spektrum einer erfindungsgemäßen Lösung, das drei Monate nach deren Herstellung aufgenommen wurde;
Fig. 6 ein ¹⁹F-NMR-Spektrum einer erfindungsgemäßen Lösung, das sechs Monate nach deren Herstellung aufgenommen wurde; und
Fig. 7 ein ¹⁹F-NMR-Spektrum einer erfindungsgemäßen Lösung, das zwölf Monate nach deren Herstellung aufgenommen wurde.

### Materialien und Bedingungen

Zur Herstellung der erfindungsgemäßen Lösungen wurde Mannosetriflat (Hersteller: ABX GmbH, Radeberg, Deutschland) mit folgenden Eigenschaften eingesetzt, das der in Tabelle 1 angegebenen Spezifikation entsprach. Die mit "Methode" bezeichnete Spalte der Tabelle gibt das Verfahren nach dem der jeweilige Wert der Spezifikation zu bestimmen ist.

**Tabelle 1**

| Parameter | Spezifikation | Methode |
|---|---|---|
| Eigenschaften | farblose bis gelbliche nadelförmige Kristalle, geruchlos, löslich in Acetonitril | organoleptisch, visuell |
| Identität | | |
| - Spektrenvergleich | Vergleich mit Ph. Eur. und ABX-Referenzspektren | IR-Spektroscopie ¹H-NMR ¹⁹F-NMR |
| - Spezifische Drehung - | [α]_{D}²⁰ = -16.0 ± 0.8 ° (c ≥ 2.0, CHCl3) | Polarimetrie |
| - Schmelzpunkt | 119 - 122 °C | Kapillarmethode |

| Chemische Reinheit | | |
|---|---|---|
| Verunreinigungen/ähnliche Verbindungen: | | HPLC |
| -Σ(1-OH-TAM, 2-OH-TAM) | < 0.2 % | |
| - jede einzelne sonstige Verunreinigung | < 0.1 % | |
| - Summe aller sonstigen Verunreinigungen | < 0.5 % | |
| Fluorhaltige Verunreinigungen: | | ¹⁹F-NMR |
| - Trifluoromethansulfonsäure | < 0.2 % | |
| - jede einzelne sonstige fluorhaltige Verunreinigung | < 0.1 mol-% | |
| - Summe aller sonstigen fluorhaltigen Verunreinigungen | < 0.3 mol-% | |

| Restlösungsmittel: | | |
|---|---|---|
| - Cyclohexan | < 0.2500 % | GC |
| - CH₂Cl₂ | < 0.1800 % | GC |
| - Pyridin | < 0.0100 % | GC |
| - Ethanol | < 0.1000 % | GC |
| - Trockenverlust | < 0.60 % | TG |
| Substanz chemisch rein | C 37.50 = 0.30 % | Elementaranalyse |
| | H 3.99 ± 0.30 % | |
| | S 6.68 ± 0.30 % | |
| Gehalt (HPLC) | 95 % - 105 % | HPLC |
| Mikrobiologische Reinheit der Bulk-Substanz | steril | Sterilität Ph. Eur. 2.6.1 |
| | Bakterien Endotoxine < 1.000 I.E./mg | LAL-Test (Ph. Eur. 2.6.14, Methode C) |

| | | |
|---|---|---|
| (GC = Gaschromatographie; TG = Thermogravimetrie; 1. E. = International Unit: substanzspezifische Einheit zur Wirksioftgehail-Standardisierung, Angaben in % in Tabelle 1 sind als Gew.-% zu verstehen, sofern nichts anderes angegeben ist; Angaben in mol-%. Zur Ermittlung der Mol-% werden die nach Integration der Peaks erhaltene Signalintensitaeten im 1H-NMR (welche direkt die Mol-Anteile repräsentieren) in eine Gleichung zur Berechnung des Molenbruchs eingesetzt und das Ergebnis mit 100 multipliziert.) | | |

Das als Lösungsmittel verwendete Acetonitril (Hersteller: Merck KGaA, Darmstadt, Deutschland) wies folgende Eigenschaften auf: Reinheit: mindestens 99,9 %, Wassergehalt: höchstens 0,05 Gew.-%.

Das als Lösungsmittel verwendete deuterierte Acetonitril (Hersteller: Fluka, Buchs, Schweiz) wies folgende Eigenschaften auf: Isotopenanreicherung: 99,8 %, Wassergehalt: höchstens 0,05 Gew.-%.

Das Lösen wurde bei Raumtemperatur, einer Luftfeuchtigkeit von 60 ± 20 % und einer Partikelkonzentration in der Umgebungsluft, die den Vorgaben der Reinraumklasse C gemäß der GMP-Richtlinie entsprach, durchgeführt.

Die Mikrofiltration wurde bei Raumtemperatur, einer Luftfeuchtigkeit von 60 ± 20 % und einer Partikelkonzentration in der Umgebungsluft, die den Vorgaben der Reinraumklasse A gemäß der GMP-Richtlinie entsprach, durchgeführt.

### Beispiel 1

40 mg Mannosetriflats wurden in 0,7 ml Acetonitril gelöst und über einen Mikrofilter (0,2 µm) filtriert. Die so hergestellte Lösung wurde in gereinigte und γ-sterilisierte Klarglasvials unter Argonatmosphäre abgefüllt und mit einem Flurotec®beschichteten Butylgummistopfen verschlossen und vercrimpt. Die so hergestellte Lösung wurde bei Raumtemperatur gelagert. ¹⁹F-NMR-Analysen zeigten, daß die Lösung eine Lagerstabilität von mehr als einem Jahr aufwies.

In diesem Zeitraum ist der Einsatz zur [¹⁸F.]FDG-Synthese gut möglich und man erhält dabei [¹⁶F]FDG-Ausbeuten von ca. 50 %.

### Beispiel 2

40 mg Mannosetriflats wurden in 0,7 ml Deuteroacetonitril gelöst und über einen Mikrofilter (0,2 µm) filtriert. Die so hergestellte Lösung wurde in gereinigte und γ-sterilisierte Klarglasvials unter Argonatmosphäre abgefüllt und mit einem Flurotec^{®}-beschichteten Chlorbutylgummi verschlossen und vercrimpt. Die so hergestellte Lösung zeigt bei Raumtemperatur nach einem Jahr kein Triflat als Zersetzungsprodukt.

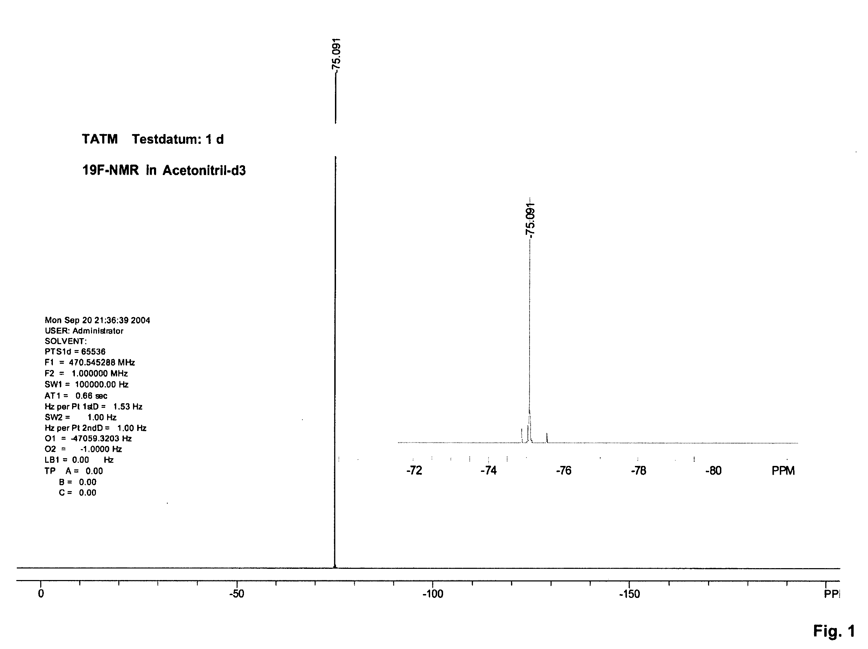

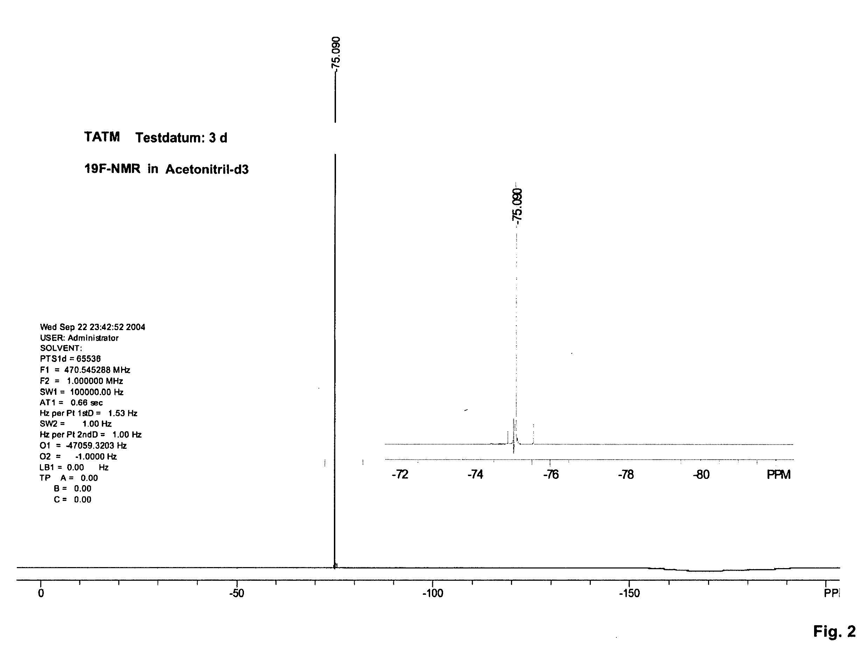

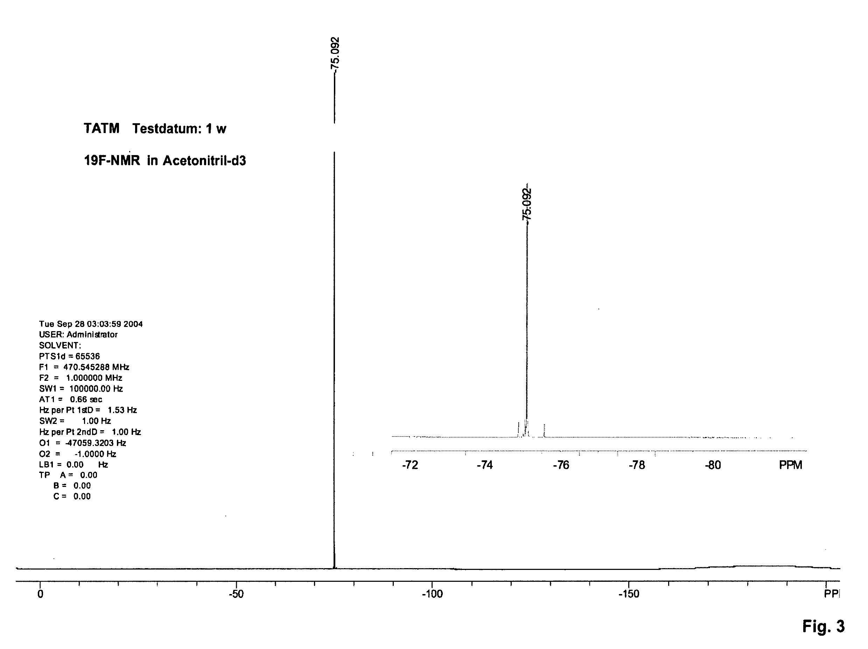

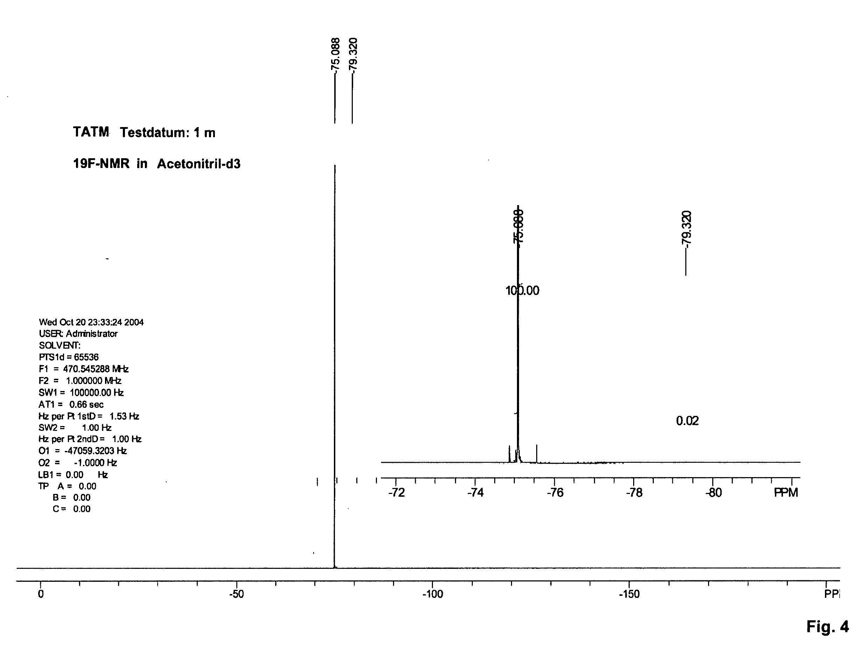

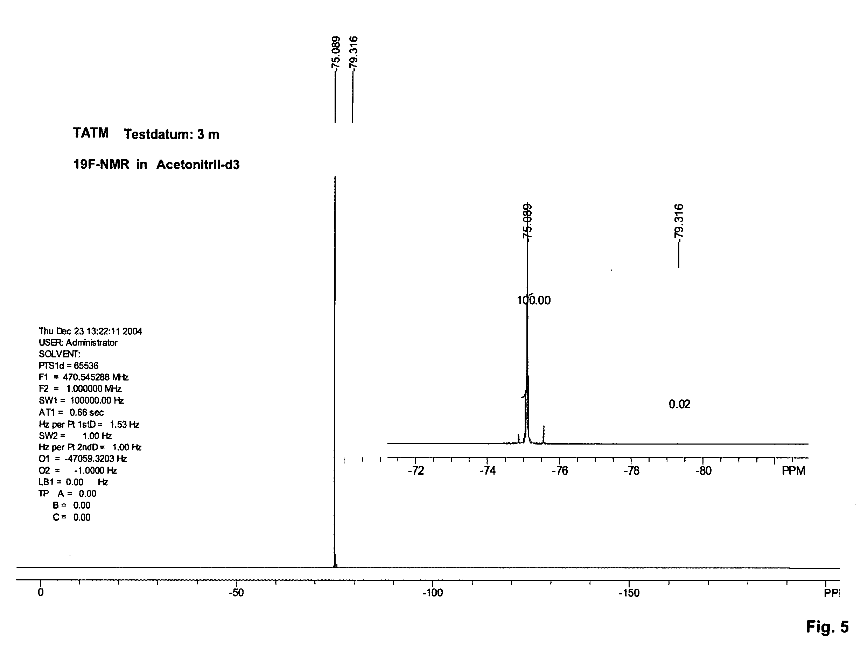

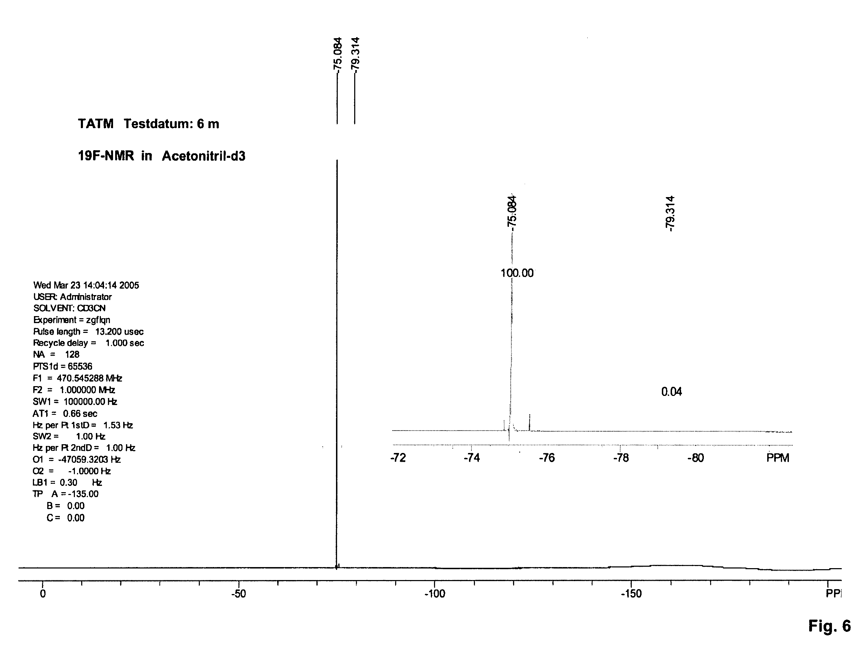

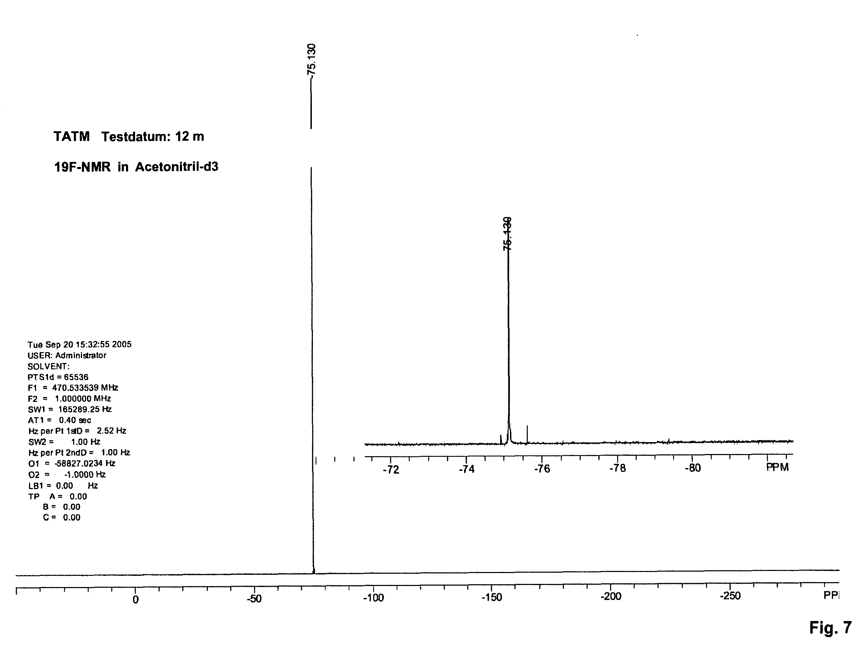

Die in den Figuren 1 bis 7 dargestellten ¹⁹F-NMR-Spektren zeigen, daß die Lösung eine Lagerstabilität von mehr 1 Jahr aufwies.

### Beispiel 3

65 mg Mannosetriflats wurden in 0,7 ml Acetonitril gelöst und über einen Mikrofilter (0,2 µm) filtriert. Die so hergestellte Lösung wurde in gereinigte und γ-sterilisierte Klarglasvials unter Argonatmosphäre abgefüllt und mit einem Flurotec®beschichteten Butylgummistopfen verschlossen und vercrimpt. Die so hergestellte Lösung wurde bei Raumtemperatur gelagert. ¹⁹F-NMR-Analysen zeigten, daß die Lösung eine Lagerstabilität von mehr 1 Jahr aufwies.

In diesem Zeitraum ist der Einsatz zur [¹⁸F]FDG-Synthese gut möglich und man erhält dabei [¹⁸F]FDG-Ausbeuten von ca. 50 %.

### Beispiel 4

25 mg Mannosetriflats wurden in 0,7 ml Acetonitril gelöst und über einen Mikrofilter (0,2 µm) filtriert. Die so hergestellte Lösung wurde in gereinigte und γ-sterilisierte Klarglasvials unter Argonatmosphäre abgefüllt und mit einem Flurotec®beschichteten Butylgummistopfen verschlossen und vercrimpt. Die so hergestellte Lösung wurde bei Raumtemperatur gelagert. ¹⁹F-NMR-Analysen zeigten, daß die Lösung eine Lagerstabilität von mehr 1 Jahr aufwies.

In diesem Zeitraum ist der Einsatz zur [¹⁸F] FDG-Synthese gut möglich und man erhält dabei [¹⁸F]FDG-Ausbeuten von ca. 50 %.

## Patentansprüche

1. Verfahren zur Herstellung einer Lösung einer Verbindung gemäß Formel (I) 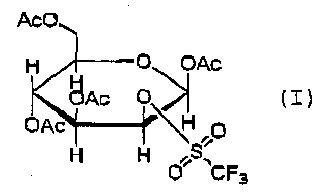 in einem Lösungsmittel, das aus der Gruppe gewählt ist, die Acetonitril, deuteriertes Acetonitril sowie Gemische dieser umfaßt, umfassend die Schritte
(a) Bereitstellen der Verbindung gemäß Formel (I), mit einer Reinheit von zumindest 99,0 Gew.-%, wobei der Anteil an Trifluormethansulfonsäure 0,2 Gew.-% nicht übersteigen darf.
(b) Bereitstellen des Lösungsmittels mit einer Reinheit von 99,9 Gew.-%, wobei der Wassergehalt des Lösungsmittels 0,05 Gew.-% nicht übersteigen darf;
(c) das Lösen der Verbindung gemäß Formel (I) in dem Lösungsmittel bei einer Luftfeuchtigkeit von höchstens 60 % und einer Raumtemperatur von höchstens 30 °C;
(d) das Mikrofiltrieren der in Schritt (c) erhaltenen Lösung über einem Mikrofilter mit einer Porengröße von höchstens 0,3 µm; und
(e) das Abfüllen der in Schritt (c) erhaltenen Lösung in zumindest einen Behälter aus einem inerten Material und Verschließen des Behälters mit einem Verschluß aus demselben oder einem anderen inerten Material.

2. Verfahren nach Anspruch 1, **dadurch gekennzeichnet, daß** die in Schritt (a) bereitgestellte Verbindung der Formel I höchstens 0,2 Gew.-% von Verunreinigungen enthält, die zu der Gruppe gehören, die aus 2, 3, 4, 6-Tetra-*O-*acetyl-β-D-mannopyranose, 1, 3, 4, 6-Tetra-*O*-acetyl-β-D-mannopyranose und Gemischen dieser besteht.

3. Verfahren nach Anspruch 1 oder Anspruch 2, **dadurch gekennzeichnet, daß** die in Schritt (a) bereitgestellte Verbindung der Formel I höchstens 0,2 Gew.-% Trifluormethansulfonsäure enthält.

4. Verfahren nach einem der vorstehenden Ansprüche, **dadurch gekennzeichnet, daß** Schritt (c) in der Umgebungsluft durchgeführt wird, die höchstens 350.000 Partikel mit einer Größe von 0,5 µm oder größer pro Kubikmeter Umgebungsluft und höchstens 2.000 Partikel mit einer Größe von 5 µm oder größer pro Kubikmeter Umgebungsluft enthält.

5. Verfahren nach einem der vorstehenden Ansprüche, **dadurch gekennzeichnet, daß** Schritt (d) in der Umgebungsluft durchgeführt wird, die höchstens 3500 Partikel mit einer Größe von 0,5 µm oder größer pro Kubikmeter Umgebungsluft und keine Partikel mit einer Größe von 5 µm oder größer pro Kubikmeter Umgebungsluft enthält.

6. Verfahren nach einem der vorstehenden Ansprüche, **dadurch gekennzeichnet, daß** der Behälter, in denen die Lösung gemäß Schritt (e) abgefüllt wird, zuvor mittels γ-Strahlung sterilisiert worden ist.

7. Verfahren nach einem der vorstehenden Ansprüche, **dadurch gekennzeichnet, daß** der Behälter ein Glasbehälter ist.

8. Verfahren nach einem der vorstehenden Ansprüche, **dadurch gekennzeichnet, daß** der Behälter aus Glas der hydrolytischen Glasklasse 1 gemäß DIN EN ISO B362-1 besteht.

9. Verfahren nach einem der vorstehenden Ansprüche, **dadurch gekennzeichnet, daß** der Verschluß des Behälters mit dem inerten Material ummantelt ist.

10. Verfahren nach einem der vorstehenden Ansprüche, **dadurch gekennzeichnet, daß** die in die Behälter abgefüllte Lösung eine Lagerstabilität von mindestens einem Jahr bei Raumtemperatur aufweist.

11. Lösung einer Verbindung gemäß Formel (I) 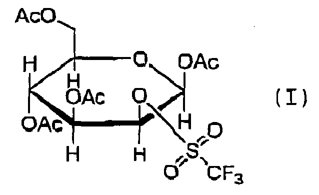 in einem Lösungsmittel, das aus der Gruppe gewählt ist, die Acetonitril, deuteriertes Acetonitril sowie Gemische dieser umfaßt, hergestellt durch das Verfahren nach einem der Ansprüche 1 bis 9.

12. Verwendung der Lösung gemäß Anspruch 11 zur Synthese von 2-[¹⁸F]Fluor-2-desoxy-D-glucose (FDG).

13. Verwendung nach Anspruch 12, **dadurch gekennzeichnet, daß** die Lösung in Mikroreaktoren oder Synthesemodulen für die Synthese von 2-[¹⁸F]Fluor-2-desoxy-D-glucose (FDG) eingesetzt wird.
